(19) Europäisches Patentamt
European Patent Office
Office européen des brevets

(11) EP 1 479 341 A1

(12) EUROPEAN PATENT APPLICATION

(43) Date of publication:
**24.11.2004 Bulletin 2004/48**

(51) Int Cl.[7]: **A61B 5/053**, G01G 19/414

(21) Application number: **04011974.5**

(22) Date of filing: **19.05.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **23.05.2003 JP 2003146842**

(71) Applicant: **TANITA CORPORATION**
**Tokyo (JP)**

(72) Inventors:
- **Shiokawa, Takashi, c/o Tanita Corp.**
  **Tokyo (JP)**
- **Kodama, Miyuki, c/o Tanita Corp.**
  **Tokyo (JP)**

(74) Representative: **Grünecker, Kinkeldey,**
**Stockmair & Schwanhäusser Anwaltssozietät**
**Maximilianstrasse 58**
**80538 München (DE)**

(54) **Muscle measuring apparatus**

(57) A body fat mass and a fat free mass are calculated by BIA, and a muscle volume is calculated by subtracting bone mineral calcium from the fat free mass. The calculated muscle volume is normalized by a value using the body height of a subject, and a muscle volume per body part is calculated. The level of the muscle volume is determined by comparing the muscle volume per body part with the reference value.

FIG. 1

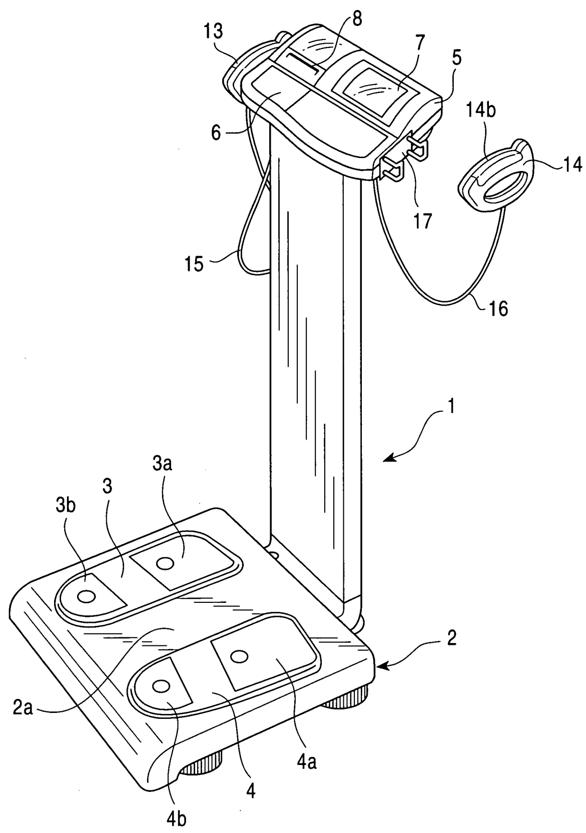


EP 1 479 341 A1

## Description

BACKGROUND OF THE INVENTION

(i) Field of the Invention

The present invention relates to an apparatus for evaluating a body composition by a bioelectrical impedance analysis in accordance with a muscle evaluating method.

(ii) Description of the Related Art

Muscles in a body are considered important due to a relationship with basal metabolism. More specifically, the more muscles a body has, the higher the basal metabolic rate of the body becomes. Thus, more intaken calories can be consumed, and the body therefore becomes less liable to gain weight. Consequently, it is believed to be preferable to increase the volume of muscles and maintain the muscles from the viewpoint of health as well. That is, knowing one's own volume of muscles is considered useful from the viewpoint of health.

An apparatus which calculates body fat by use of a bioelectrical impedance analysis (BIA) is known. Further, an apparatus which calculates a fat free mass by use of BIA, subtracts bone mineral calcium from the calculated value so as to determine the volume of muscles of a subject and displays the determined muscle volume is also known.

Further, as a method of evaluating a body composition, there is a method of calculating BMI (Body Mass Index) which is one's ratio to a standard body weight. When a body height is L (m) and a body weight is M (kg) , BMI is represented by the following expression.

$$BMI = M/L^2$$

This BMI is an index for knowing the physique of a subject and is considered useful.

An apparatus has been proposed that determines the BMI and a body fat percentage and displays the relationship between the two indices in the form of a matrix (for example, refer to Patent Publication 1).

Further, there is also an apparatus which displays FMI (Fat Mass Index: fat mass/(body height)$^2$) which is an index of fat and LMI (Lean Mass Index: fat free mass/ (body height)$^2$) which is an index of a fat free mass, together with BMI which is a body mass index (for example, refer to Patent Publication 2).

Further, there is a body fat meter which displays a body fat percentage and which calculates LBM (Lean Body Mass: fat free mass/body height) and displays whether the LBM value is large or not in terms of levels together with the LBM value. Further, the device is capable of determining a body constitution or a body shape from a combination of the LBM and the body fat percentage (for example, refer to Non-Patent Publication 1).

Patent Publication 1

Japanese Patent Publication No. 192258/1998 (pp. 2 to 4, Fig. 1)

Patent Publication 2

Japanese Patent Publication No. 125947/2002 (pp. 3 to 5, Fig. 10)

Non-Patent Publication 1

Homepage of Misaki Inc., Product Information, Body Fat Meter, BODYCHECKER C-1 [searched on May 21, 2003], Internet <URL:http://www.misaki-inc.co.jp/goods/goods_fat/c1.html>

It is considered useful in view of health to know the volume of muscles, and there is an apparatus that calculates a muscle volume excluding bone mineral calcium by use of BIA in a conventional manner and displays the muscle volume. However, the apparatus displays the measured muscle volume merely by numerical values, and it is not easy for a subject to determine whether the muscle volume is a proper value. Further, since such an apparatus which displays a muscle volume by numerical values uses an amount as an index for the determination, a difference in muscle volume due to a difference in body height occurs. That is, those who are tall have large muscle volumes on the whole regardless of their physiques, and their muscle volumes cannot be compared with the muscle volumes of those who are short in body height on the same basis.

Further, the apparatus described in the above Japanese Patent Publication No. 192258/1998 merely displays a combination of BMI and a body fat percentage and does not directly make an evaluation on muscles.

Further, in the case of conventional apparatuses which evaluate muscles, a fat free mass (FFM) including liquids such as blood and an extracellular fluid and bones is often used as a value approximate to a muscle volume. Likewise, the above body fat meter which calculates LBM also uses a fat free mass, and it makes an evaluation by normalizing (dividing) the value of the fat free mass by a body height. When the fat free mass is treated as a value approximate to a muscle volume, accurate data on the muscle volume cannot be obtained since a change in bone tissue (bone mineral calcium) is different from a change in muscular tissue (muscle volume). That is to say, when the change in muscle volume is examined, the change in bone mineral calcium interferes with it, resulting in inaccurate data. Further, when the proportion of muscle volume in a fat free mass is small, the proportion of bone mineral calcium which is a cause of an error becomes large, so that an accurate evaluation of the muscle volume cannot be made.

In addition, even if a body shape is determined from a matrix of two indices, i.e., LBM and a body fat percentage, the accuracy of the determination of the body shape is in question, because the LBM, in the first place, is a value obtained by dividing a fat free mass including bone mineral calcium by a body height and is not a value reflecting only a muscle volume.

Such problems in using a fat free mass as an index of a muscle volume can also be mentioned with respect to the apparatus described in the above Japanese Patent Publication No. 125947/2002, and it can hardly be said that the apparatus makes an accurate evaluation of muscles.

The present invention has been conceived in view of the above problems. An object of the present invention is to make a more accurate evaluation of the volume of muscles in measurement of the muscle volume and show if the muscle volume of a subject is large or not in an easily understandable manner by making the result of the evaluation easily understandable to the subject. Further, another object of the present invention is to make an overall evaluation on the body of a subject by combining a muscle volume with other physical index (indices).

SUMMARY OF THE INVENTION

A muscle measuring apparatus of the present invention comprises:

an input unit,
a bioelectrical impedance measuring unit,
a muscle volume calculating unit, and
a muscle level calculating unit,

wherein
the input unit is used to input personal physical data of a subject which includes at least a body height,
the bioelectrical impedance measuring unit measures a bioelectrical impedance,
the muscle volume calculating unit calculates the volume of muscles of the subject based on the personal physical data entered from the input unit and the measured bioelectrical impedance value, and
the muscle level calculating unit calculates the level of the muscle volume of the subject based on a muscle volume per reference unit which is calculated by "muscle volume/(body height)$^n$ (n is a real number)" using the body height of the subject and the calculated muscle volume. The apparatus evaluates the measured muscle volume in terms of levels.

Further, in the muscle measuring apparatus of the present invention, the muscle level calculating unit calculates the level of the muscle volume as zero when the muscle volume is a standard value, as a negative level when the muscle volume is small, and as a positive level when the muscle volume is large, thereby making it easy for a subject to know his/her muscle volume.

The muscle measuring apparatus of the present invention further comprises:

a body fat calculating unit, and
a somatotype determining unit,

wherein
the body fat calculating unit calculates the body fat percentage of the subject based on the physical data entered from the input unit and the measured bioelectrical impedance value, and the somatotype determining unit makes a determination with respect to a body shape or a body constitution based on a combination of the body fat and the muscle level. Thereby, the apparatus makes an evaluation on the overall body shape of a subject.

The muscle measuring apparatus of the present invention further comprises:

a body weight measuring unit,
a BMI calculating unit, and
a muscle determining unit,

wherein
the body weight measuring unit measures the body weight of the subject,
the BMI calculating unit calculates BMI from the body height entered from the input unit and the measured body weight value, and
the muscle determining unit determines the volume of muscles in the BMI of the subject based on a combination of the muscle level and the BMI. Thereby, the apparatus evaluates a muscle volume from different angles.

BRIEF DESCRIPTION OF THE DRAWINGS

Fig. 1 is an external view of a body composition measuring apparatus in an embodiment of the present invention.
Fig. 2 is an internal block diagram of the body composition measuring apparatus in the embodiment of the present invention.
Fig. 3 is a flowchart of the body composition measuring apparatus in the embodiment of the present invention.
Fig. 4 is a diagram showing the results of calculations of a body weight, a body fat mass and a body fat percentage in the embodiment of the present invention.
Fig. 5 is a matrix evaluation table based on the relationship between a muscle score and a body fat percentage in the embodiment of the present invention.

Fig. 6 is an evaluation table based on the relationship between a muscle score and BMI in the embodiment of the present invention.

Fig. 7 is a diagram showing the results of measurement/calculation/determination made with respect to muscles in the embodiment of the present invention.

Fig. 8 is a diagram showing muscle age in the embodiment of the present invention.

Fig. 9 is a diagram showing overall results in the embodiment of the present invention.

## DETAILED DESCRIPTION OF THE PREFERRED EMBODIMENTS

In the muscle measuring apparatus of the present invention, a body fat mass and a fat free mass are calculated by BIA, and a muscle volume is calculated by subtracting bone mineral calcium from the fat free mass. The calculated muscle volume is normalized by a value using the body height of a subject, and a muscle volume per body part is calculated. The level of the muscle volume is determined by comparing the muscle volume per body part with the reference value.

The level of the muscle volume is determined to be a zero level when the muscle volume is a standard volume, a positive level when the muscle volume is large, and a negative level when the muscle volume is small.

Further, the apparatus makes an evaluation with respect to the body shape or body constitution of the subject based on the relationship between the level of the muscle volume and a body fat percentage. Further, the apparatus determines the volume of muscles in the BMI of the subject.

Examples of the present invention will be described with respect to the drawings.

Fig. 1 is an external view of a body composition measuring apparatus. Fig. 2 is a block diagram illustrating electrical connections of the apparatus.

Fig. 1 is an external perspective view of the body composition measuring apparatus which is an embodiment of the present invention. The measuring apparatus 1 is nearly L-shaped. The apparatus 1 has a scale 2 in the lower portion. The scale 2 is a known device and has a platform 2a on which a subject stands so as to measure his/her body weight. On the platform 2a, electrodes 3 and 4 which make contact with the bottoms of both feet of a subject are provided. The electrodes 3 and 4 comprise current supplying electrodes 3a and 4a for supplying an electric current and voltage measuring electrodes 3b and 4b for measuring a voltage.

Further, on the top surface of the measuring apparatus 1, an operation box 5 is provided. This operation box 5 comprises an input unit 6 which is input means used to input various physical data and comprises a plurality of keys including a power switch and numerical keys, a display unit 7 which is display means comprising a dot matrix LCD for displaying the results of measurements, and a printing unit 8 which prints the results of measurements on paper and ejects the paper.

Further, to the operation box 5, electrode grips 13 and 14 for hands are connected via cords 15 and 16. The electrode grips 13 and 14 comprise current supplying electrodes 13a and 14a for supplying an electric current and voltage measuring electrodes 13b and 14b for measuring a voltage. The electrode grips 13 and 14 are kept hooked on hooks 17 which are provided on both sides of the operation box 5, except for when they are used for measurement.

Fig. 2 is an internal electrical block diagram of the measuring apparatus 1. Eight electrodes which are current applying means and voltage measuring means, i.e., electrodes 3a, 3b, 4a, 4b, 13a, 13b, 14a and 14b which make contact with both hands and feet, are connected to an electrode switching unit 20. The electrode switching unit 20 is connected to a arithmetic and control unit 23 which is control means via a current supplying unit 21 and a voltage measuring unit 22. The arithmetic and control unit 23 has a microcomputer (CPU) and is not only bioelectrical impedance measuring means for calculating a bioelectrical impedance from an applied electric current and a measured voltage but also muscle volume calculating means for calculating a muscle volume and muscle level calculating means for calculating the level of the muscle volume. Further, the arithmetic and control unit 23 is also body fat calculating means for calculating the body fat percentage and body fat mass of a living body, BMI calculating means for calculating BMI and muscle evaluating means for evaluating the relationship of a muscle volume with BMI and also performs various other computations and controls. A storage unit 24 which is storage means for storing various data and comprises a memory or a register and a body weight measuring unit 26 which measures the body weight of a subj ect are connected to the arithmetic and control unit 23. Further, the input unit 6, the display unit 7 and the printing unit 8 are also connected to the arithmetic and control unit 23. A power unit 28 supplies electric power to the arithmetic and control unit 23 and other units.

Next, the operation of the body composition measuring apparatus will be described.

Fig. 3 is a flowchart illustrating the operation of the body composition measuring apparatus 1.

At the press of the power switch of the input unit 6 (STEP S1), the apparatus is initialized (STEP S2). Thereby, the apparatus enters an input mode to accept personal parameters, and the amount of clothing is set first (STEP S3). Then, a user enters personal parameters such as sex, age and a body height by use of the numerical keys of the input unit 6 (STEPS S4 to S6).

The entered personal parameters are displayed on the display unit 7, and a message for confirming whether the parameters are valid is also displayed (STEP S7). If the parameters are confirmed to be valid, the apparatus starts to make measurements, while if the parameters are invalid, the apparatus returns to STEP S3 where the amount of clothing is entered again (STEP S8).

When the personal parameters are entered, a body weight is measured (STEP S9). When the user stands on the scale 2, the body weight measuring unit 26 detects a load and measures the weight of the user.

Then, a bioelectrical impedance is measured. This measurement is made in various body parts. The measurement is made by switching body parts to which a measuring electric current is applied in turn.

Firstly, a bioelectrical impedance between both feet is measured (STEP S10). The electrode switching unit 20 is switched by a signal from the arithmetic and control unit 23, whereby an alternating electric current is supplied from the current supplying unit 21 to between the electrodes 3a and 4a, and a voltage is measured between the electrodes 3b and 4b by the voltage measuring unit 22. Then, a bioelectrical impedance Z is calculated from the applied alternating electric current value and the measured voltage value.

Similarly, a bioelectrical impedance between both hands is then measured. An alternating electric current is passed between the current supplying electrodes 13a and 14a, and a voltage is measured between the voltage measuring electrodes 13b and 14b (STEP S11).

Further, a bioelectrical impedance between the right hand and the right foot is then measured. An alternating electric current is passed between the current supplying electrodes 14a and 4a, and a voltage is measured between the voltage measuring electrodes 14b and 4b (STEP S12).

Further, a bioelectrical impedance between the left hand and the left foot is then measured. An alternating electric current is passed between the current supplying electrodes 13a and 3a, and a voltage is measured between the voltage measuring electrodes 13b and 3b (STEP S13).

Further, a bioelectrical impedance in the right leg is then measured. An alternating electric current is passed between the current supplying electrodes 14a and 4a, and a voltage is measured between the voltage measuring electrodes 3b and 4b (STEP S14).

Further, a bioelectrical impedance in the left leg is then measured. An alternating electric current is passed between the current supplying electrodes 13a and 3a, and a voltage is measured between the voltage measuring electrodes 3b and 4b (STEP S15).

Further, a bioelectrical impedance in the right arm is then measured. An alternating electric current is passed between the current supplying electrodes 14a and 4a, and a voltage is measured between the voltage measuring electrodes 13b and 14b (STEP S16).

Further, a bioelectrical impedance in the left arm is then measured. An alternating electric current is passed between the current supplying electrodes 13a and 3a, and a voltage is measured between the voltage measuring electrodes 13b and 14b (STEP S17).

After the measurements of the bioelectrical impedances in various body parts are completed, the body fat percentage of the subject is calculated (STEP S18).

Only brief descriptions will be given to methods of determining the body fat percentage, body fat mass and fat free mass of the whole body by use of bioelectrical impedances, because the methods are conventionally known techniques.

A body fat percentage "%Fat", a body fat mass "BFM" and a fat free mass "FFM" are calculated by use of a body density "BD" as follows:

$$\%Fat = (4.95/BD - 4.5) \times 100$$

$$BD = a - b \times W \times Z/H^2$$

$$BFM = W \times \%Fat/100$$

$$FFM = W \times (1 - \%Fat)/100$$

wherein W (kg) represents a body weight, H (cm) represents a body height, Z ($\Omega$) represents a measured impedance, and a and b represent a coefficient.

From these arithmetic expressions, the body fat percentage, body fat mass and fat free mass of the whole body are calculated.

Further, by comparing the bioelectrical impedances measured in the body parts and data about the body weight and the set body height with data obtained by DEXA measurement (double X-ray absorption method), a body fat percentage and a fat free mass in each body part of the limbs can be calculated. In addition, from the ratio between the body fat percentage and the fat free mass, the weight in the body part can be calculated, and a body fat mass can also be calculated.

The calculated body weight, body fat mass and body fat percentage are displayed on the display unit 7 as shown in Fig. 4 (STEP S19).

Then, a muscle volume is calculated (STEP S20). First of all, bone mineral calcium (BMC) is determined.

$$BMC = C_1 \times FFM \times C_2$$

wherein $C_1$ and $C_2$ are a constant.

Then, by the following expression which subtracts the calculated BMC from the fat free mass FFM, a muscle volume (MV) is calculated.

$$MV = FFM - BMC$$

The value calculated in accordance with the expression is the muscle volume of the subject.

Then, the level of the muscle volume of the subject is calculated from the calculated muscle volume (STEP S21).

The muscle volume of the subject is classified into different levels by a value calculated in accordance with the following expression.

$$MV/Ht^2 \ (kg/m^2)$$

This value expresses a muscle volume per unit area and tells how much muscle is present per unit surface area of the subject. This value is classified into 9 levels. As a muscle score, the middle level (level 5) is given a zero value, and the rest of the levels are given values ranging from -4 to +4 excluding 0.

Based on the value calculated as described above, a physique age level is determined as an evaluation of a physique in terms of age. It is classified into the following 9 levels according to the value of the percentage (%) of difference from actual age.

Level 1: Score -4 = less than 10 $kg/m^2$
Level 2: Score -3 = 10 $kg/m^2$ to less than 11 $kg/m^2$
Level 3: Score -2 = 11 $kg/m^2$ to less than 12 $kg/m^2$
Level 4: Score -1 = 12 $kg/m^2$ to less than 14 $kg/m^2$
Level 5: Score 0 = 14 $kg/m^2$ to less than 16 $kg/m^2$
Level 6: Score +1 = 16 $kg/m^2$ to less than 18 $kg/m^2$
Level 7: Score +2 = 18 $kg/m^2$ to less than 19 $kg/m^2$
Level 8: Score +3 = 19 $kg/m^2$ to less than 20 $kg/m^2$
Level 9: Score +4 = 20 $kg/m^2$ or more

The above classification of the value into these levels is for the case where a subject is a male and the muscle volume of his whole body is evaluated. In this evaluation, the value is compared with a reference value set for each body part, and a muscle score is calculated for each body part.

Further, as shown in Fig. 5, a matrix is formed by two indices, i.e., a muscle score and a body fat percentage, based on the relationship between the muscle score and body fat percentage of the whole body, and the body shape and body constitution of the subject are determined. The subject can know his own body shape by determining which of these 9 groups he belongs to (STEP S22).

Further, the muscle volume of the subject from the viewpoint of BMI is evaluated based on the evaluation table shown in Fig. 6 which is based on the relationship between the BMI and the muscle score. In this table, a standard line and ±10% lines for the BMI and muscle score are shown. The standard line is a line representing a standard muscle volume at a particular BMI.

When the muscle volume is within the ±10% range, it is determined to be "normal balance which matches the BMI". When the muscle volume is above the ±10% range, it is determined to be "large muscle volume for the BMI". When the muscle volume is below the ±10% range, it is determined to be "small muscle volume for the BMI" (STEP S23).

Based on the above steps, the determined muscle volume, the muscle scores in the limbs, the result of the determination of the body shape based on the relationship between the muscle score and the body fat percentage, and the result of the determination of balance of the muscle volume based on the relationship between the muscle score and BMI are displayed on the display unit 7 as shown in Fig. 7 (STEP S24).

After passage of a certain amount of time, a graphical result showing muscle age is displayed on the display unit 7 as shown in Fig. 8. This value is muscle age representing the calculated value of the muscle volume/(body height)$^2$ of the subject and is displayed together with a line formed by connecting average values at various ages. It is possible to determine what age the muscle age of the subject corresponds to, based on the relationship between the subject's value and the average values at various ages.

The example of Fig. 8 represents a case where the actual age of the subject is 54. The determined muscle volume level is plotted on the axis of 54 years old. As the plotted dot is shifted left along with and parallel to the age axis, it intersects the average value line of thirties. From such a relationship, it is possible to determine which range of ages the muscle volume level of the subject corresponds to.

Then, overall results are displayed. As shown in Fig. 9, results regarding muscles, together with the results of measurements of the body weight and body fat, are displayed on the display unit 7.

At this point, when a print switch on the input unit 6 is pressed, the measurement results are printed on paper in the printing unit 8, and the paper is then ejected from the unit 8 (STEPS S25 and S26).

If a certain amount of time is elapsed without the print switch being pressed in STEP S25, the apparatus is shut off, and the whole procedure is completed (STEP S27).

In the above description, an embodiment of the present invention has been described in such a way that muscle volumes in various body parts are measured by an eight electrode method using eight electrodes. However, the present invention is applicable to an apparatus which makes a measurement only between both hands or only between both feet if arithmetic expressions corresponding to various body parts are used, and body parts to be measured are not limited.

Further, in the above description, it has been described that the body height of the subject is entered by use of the switches. Alternatively, it is also possible that the muscle measuring apparatus of the present invention is connected to a body height meter so as to take in body height data automatically and use the data as input data.

Further, it has been described above that when n = 2 in muscle volume/(body height)$^n$ for determination of the muscle level, a muscle volume per unit area is used to determine the muscle level. However, the value of n is not limited to the value and may be any real number. For example, when n = 1, a muscle volume per unit length is used, and when n = 3, a muscle volume per unit volume is used. Regardless of the value of n, the above expression represents a muscle volume per reference unit based on a body height, and the value of n is not limited.

Further, it has been described above as a procedure for calculating a muscle volume that a fat free mass is calculated first, bone mineral calcium is then calculated, and the bone mineral calcium is subtracted from the fat free mass so as to determine a muscle volume (MV). The procedure is not limited to this, and it is also possible that the muscle volume is determined directly from the fat free mass (FFM) as shown by the following expression:

$$MV = a_1 \times FFM + b_1$$

wherein $a_1$ and $b_1$ are a coefficient.

Alternatively, the muscle volume may also be calculated directly from the following expression using a bioelectrical impedance (Z) and a body height (Ht):

$$MV = a_2 \times Ht^2/Z + b_2$$

wherein $a_2$ and $b_2$ are a coefficient.

Further, it has also been described above that evaluations regarding the muscles of the subject are made by use of the evaluation tables shown in Figs. 5 and 6. However, it is also possible to display the evaluation tables on the display unit and visualize which range the subject belongs to.

Further, it has also been described above that the result of an evaluation of muscles is displayed by use of the muscle score. However, it is also acceptable to display the result not by use of such numerical values but by use of words that the subject can image easily. For example, it is conceivable to display "amateur level" when a muscle volume is small and display such expressions as "semiprofessional level" and "professional level" as the muscle volume increases.

Alternatively, it is also possible to evaluate an appropriate exercise type of the subject from distribution of muscle volumes in various body parts as an evaluation of the muscle volumes. For example, the result of the evaluation may be displayed by use of such an expression as "throwing event type" when the subject has a large amount of muscles in the upper body or "runner type" when the subject has a large amount of muscles in the lower body. Alternatively, a specific sport which is suitable for the subject may be displayed. For example, when the subj ect has a large amount of muscles in the upper body, "javelin throwing or shot-putting" is displayed; when the subject has a large amount of muscles in the lower body, "sprint" is displayed; and when the subject has a large amount of muscles in the whole body, "triathlon" is displayed.

In the muscle measuring apparatus of the present invention, a more accurate muscle volume is calculated by subtracting bones and liquids such as an extracellular fluid from a fat free mass, and the calculated muscle volume is displayed by levels. Thereby, it becomes easy to know one's own muscles which have heretofore been difficult to know, and this can be used for health management.

Further, in the muscle measuring apparatus of the present invention, the measured muscle volume of a subject is displayed as a zero level when the muscle volume is a standard value, as a positive level when the muscle volume is large, and as a negative level when the muscle volume is small. Thereby, it becomes easy to know one's own muscle volume, and health can be managed more easily.

Further, the muscle measuring apparatus of the present invention makes a determination based on a muscle volume level and a body fat percentage. Thus, the body shape or body constitution of subject such as "muscular obese type" who has not only a large amount of muscles but also a large amount of body fat or "slim type" who has a low body fat percentage and a small amount of muscles can be known, and this can be used for better health management.

Further, the muscle measuring apparatus of the present invention makes a determination based on the relationship between a muscle volume level and BMI. Since the BMI which is a global standard for determination of the degree of obesity is determined based only on the relationship between a body weight and a body height, athletes having a large amount of muscles may be determined to be obese. Thus, by making a determination based on the relationship between the BMI and the muscle volume level, a balance between a muscle volume and a fat mass can be known, and this can be used for better health management.

## Claims

**1.** A muscle measuring apparatus comprising:

an input unit,
a bioelectrical impedance measuring unit,
a muscle volume calculating unit, and
a muscle level calculating unit,

wherein

the input unit is used to input personal physical data of a subject which includes at least a body height,
the bioelectrical impedance measuring unit measures a bioelectrical impedance,
the muscle volume calculating unit calculates the volume of muscles of the subject based on the personal physical data entered from the input unit and the measured bioelectrical impedance value, and
the muscle level calculating unit calculates the level of the muscle volume of the subject based on a muscle volume per reference unit which is calculated by "muscle volume/(body height)$^n$ (n is a real number)" using the body height of the subject and the calculated muscle volume.

**2.** The apparatus of claim 1, wherein the muscle level calculating unit calculates the level of the muscle volume as zero when the muscle volume is a standard value, as a negative level when the muscle volume is small, and as a positive level when the muscle volume is large.

**3.** The apparatus of claim 1 or 2, further comprising:

a body fat calculating unit, and
a somatotype determining unit,

wherein
the body fat calculating unit calculates the body fat percentage of the subject based on the physical data entered from the input unit and the measured bioelectrical impedance value, and
the somatotype determining unit makes a determination with respect to a body shape or a body constitution based on a combination of the body fat and the muscle level.

**4.** The apparatus of claim 1 or 2, further comprising:

a body weight measuring unit,
a BMI calculating unit, and
a muscle determining unit,

wherein
the body weight measuring unit measures the body weight of the subject,
the BMI calculating unit calculates BMI from the body height entered from the input unit and the measured body weight value, and
the muscle determining unit determines the volume of muscles in the BMI of the subject based on a combination of the muscle level and the BMI.

FIG. 1

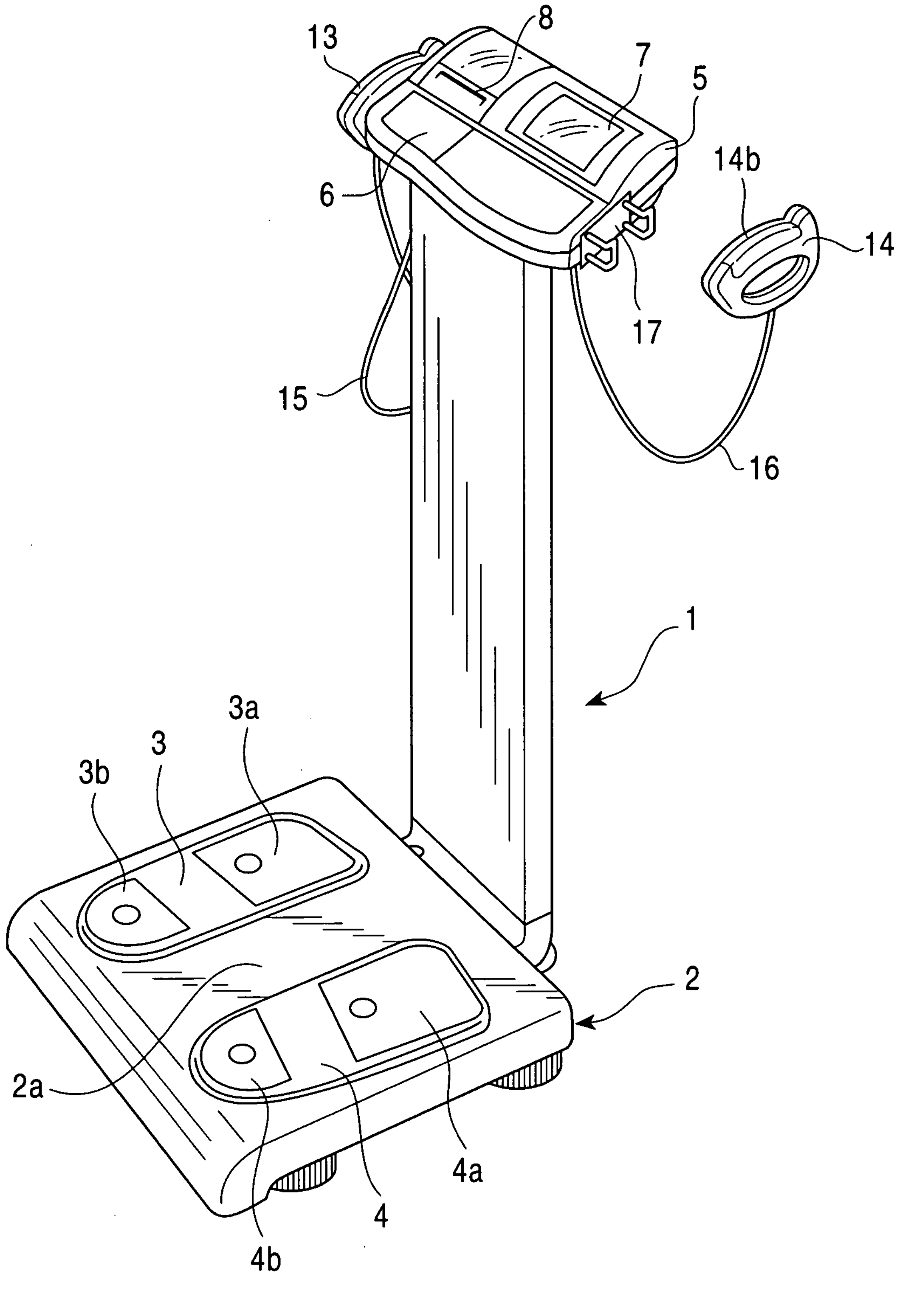

FIG. 2

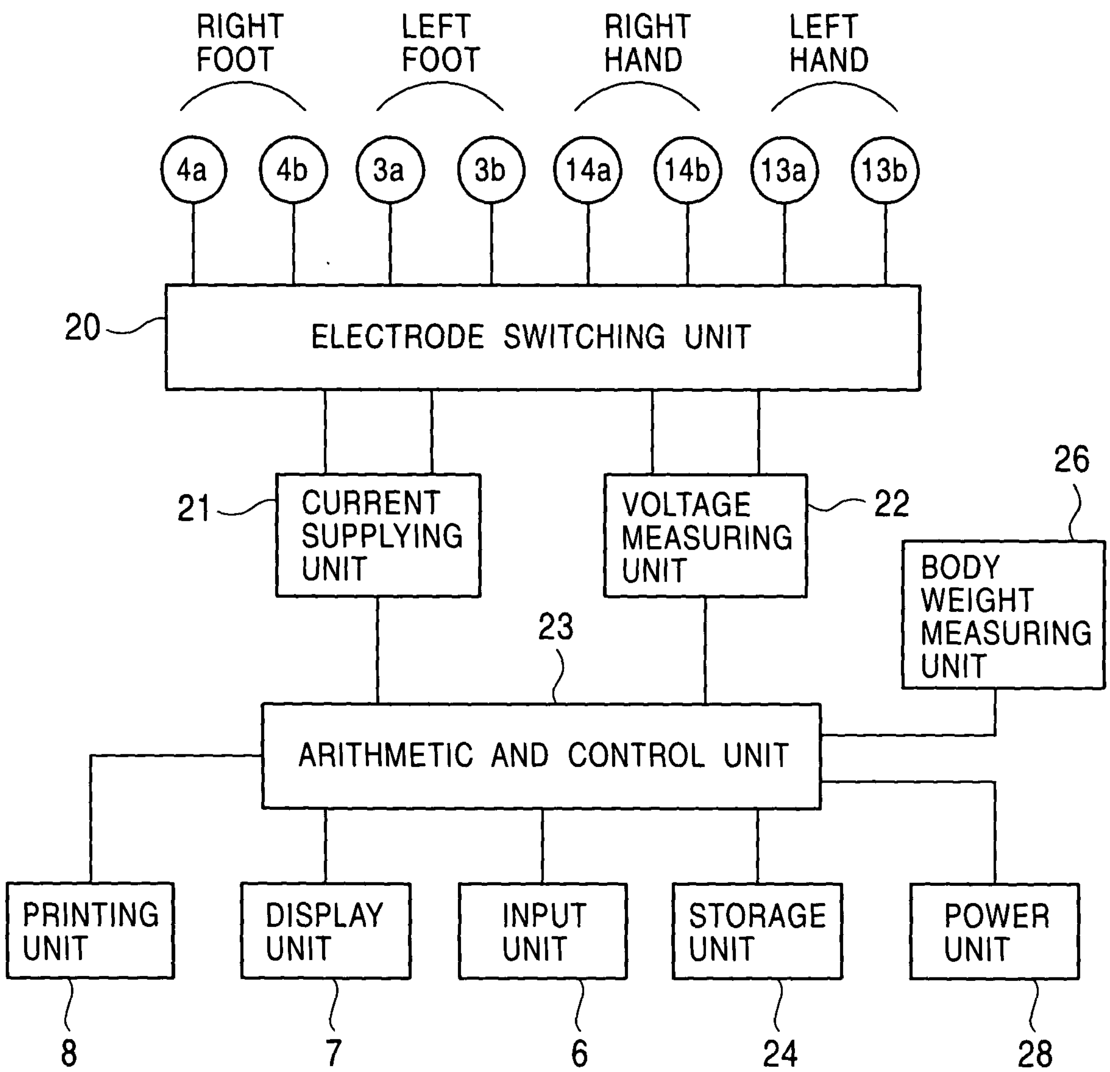
RIGHT FOOT
LEFT FOOT
RIGHT HAND
LEFT HAND
4a
4b
3a
3b
14a
14b
13a
13b
20
ELECTRODE SWITCHING UNIT
21
CURRENT SUPPLYING UNIT
VOLTAGE MEASURING UNIT
22
26
BODY WEIGHT MEASURING UNIT
23
ARITHMETIC AND CONTROL UNIT
PRINTING UNIT
DISPLAY UNIT
INPUT UNIT
STORAGE UNIT
POWER UNIT
8
7
6
24
28

FIG. 3

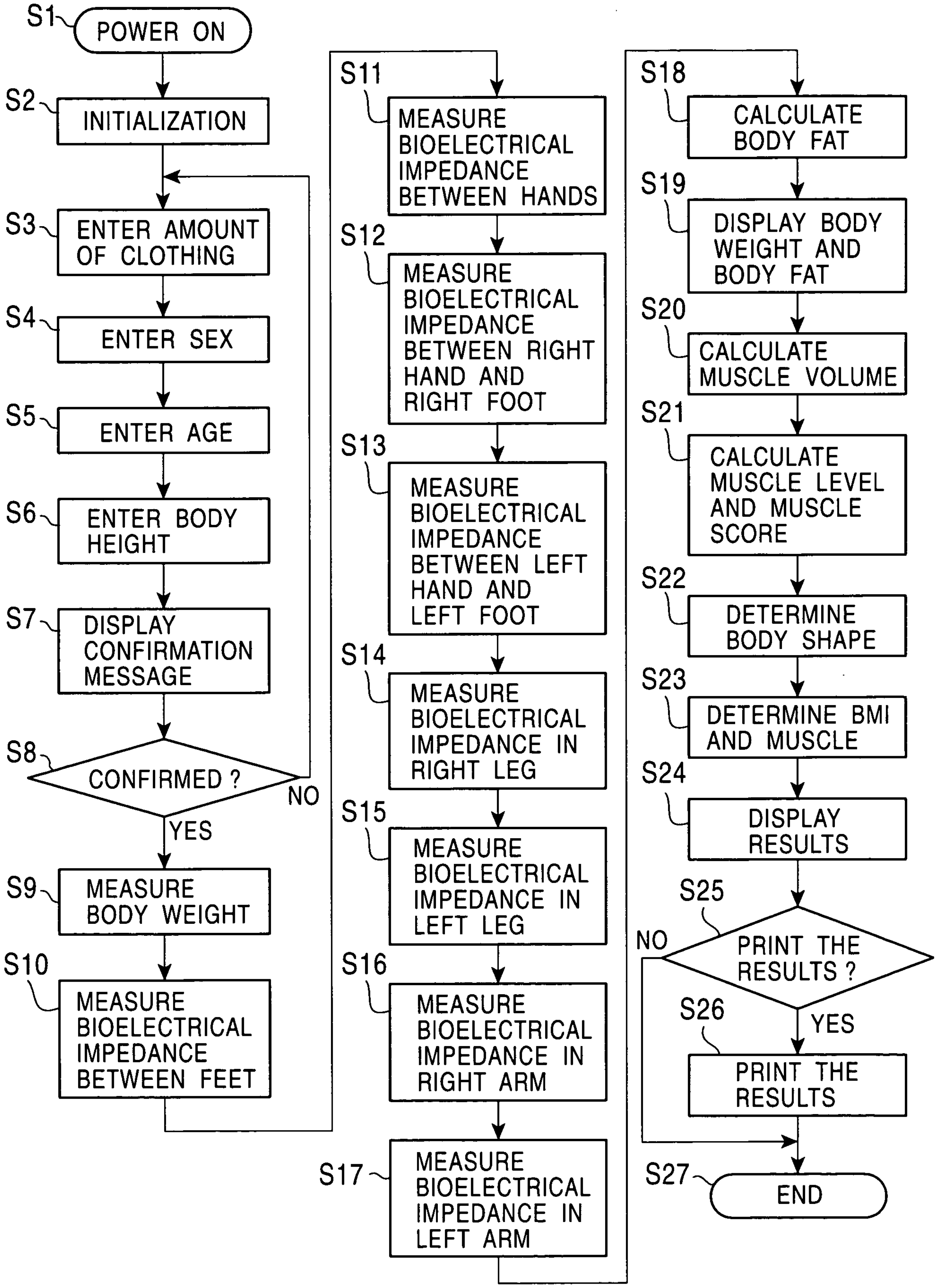
S1
POWER ON
S2
INITIALIZATION
S3
ENTER AMOUNT OF CLOTHING
S4
ENTER SEX
S5
ENTER AGE
S6
ENTER BODY HEIGHT
S7
DISPLAY CONFIRMATION MESSAGE
S8
CONFIRMED ?
NO
YES
S9
MEASURE BODY WEIGHT
S10
MEASURE BIOELECTRICAL IMPEDANCE BETWEEN FEET
S11
MEASURE BIOELECTRICAL IMPEDANCE BETWEEN HANDS
S12
MEASURE BIOELECTRICAL IMPEDANCE BETWEEN RIGHT HAND AND RIGHT FOOT
S13
MEASURE BIOELECTRICAL IMPEDANCE BETWEEN LEFT HAND AND LEFT FOOT
S14
MEASURE BIOELECTRICAL IMPEDANCE IN RIGHT LEG
S15
MEASURE BIOELECTRICAL IMPEDANCE IN LEFT LEG
S16
MEASURE BIOELECTRICAL IMPEDANCE IN RIGHT ARM
S17
MEASURE BIOELECTRICAL IMPEDANCE IN LEFT ARM
S18
CALCULATE BODY FAT
S19
DISPLAY BODY WEIGHT AND BODY FAT
S20
CALCULATE MUSCLE VOLUME
S21
CALCULATE MUSCLE LEVEL AND MUSCLE SCORE
S22
DETERMINE BODY SHAPE
S23
DETERMINE BMI AND MUSCLE
S24
DISPLAY RESULTS
S25
PRINT THE RESULTS ?
NO
YES
S26
PRINT THE RESULTS
S27
END

FIG. 4

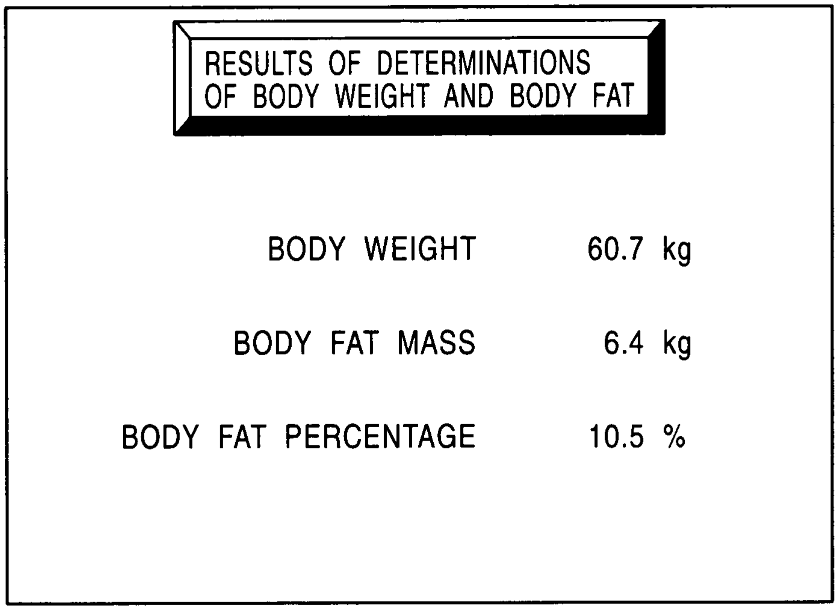
RESULTS OF DETERMINATIONS
OF BODY WEIGHT AND BODY FAT
BODY WEIGHT 60.7 kg
BODY FAT MASS 6.4 kg
BODY FAT PERCENTAGE 10.5 %

FIG. 5

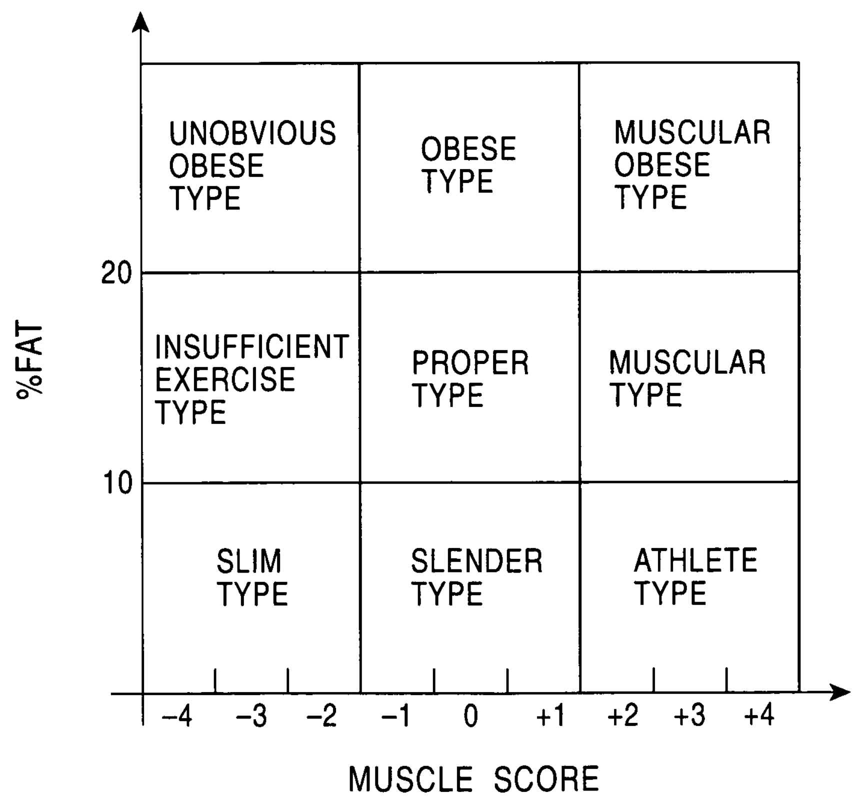
UNOBVIOUS OBESE TYPE
OBESE TYPE
MUSCULAR OBESE TYPE
INSUFFICIENT EXERCISE TYPE
PROPER TYPE
MUSCULAR TYPE
SLIM TYPE
SLENDER TYPE
ATHLETE TYPE
%FAT
20
10
−4 −3 −2 −1 0 +1 +2 +3 +4
MUSCLE SCORE

FIG. 6

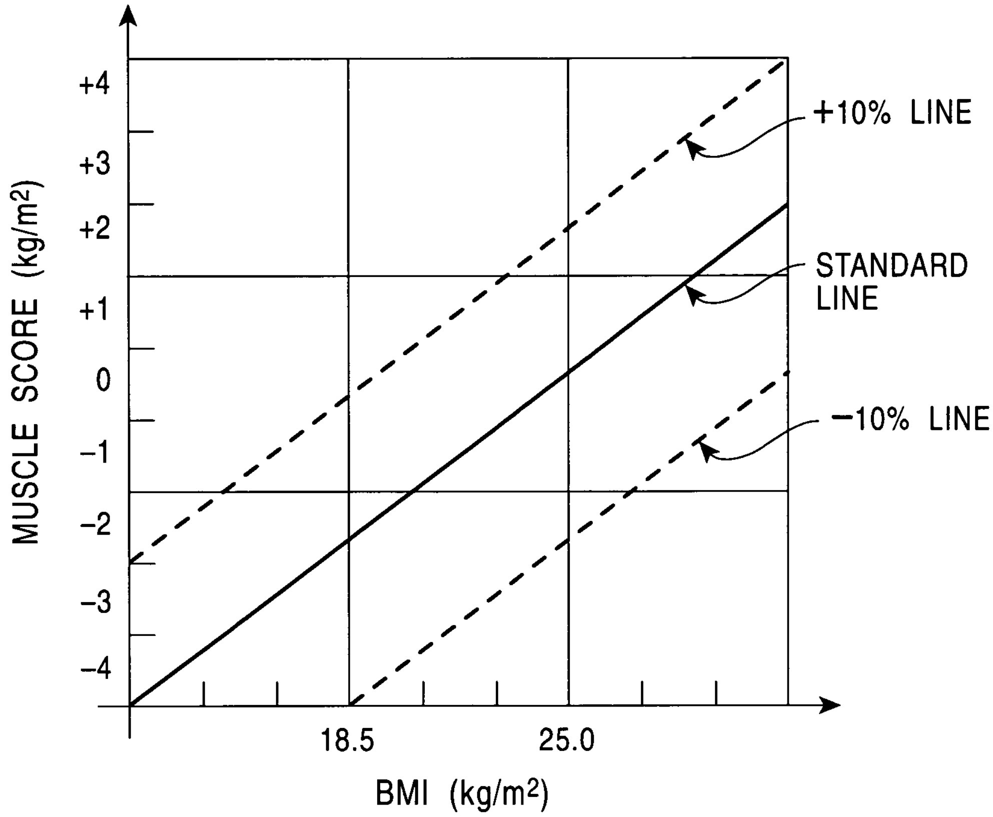
MUSCLE SCORE (kg/m2)
+4
+3
+2
+1
0
−1
−2
−3
−4
18.5
25.0
BMI (kg/m2)
+10% LINE
STANDARD LINE
−10% LINE

FIG. 7

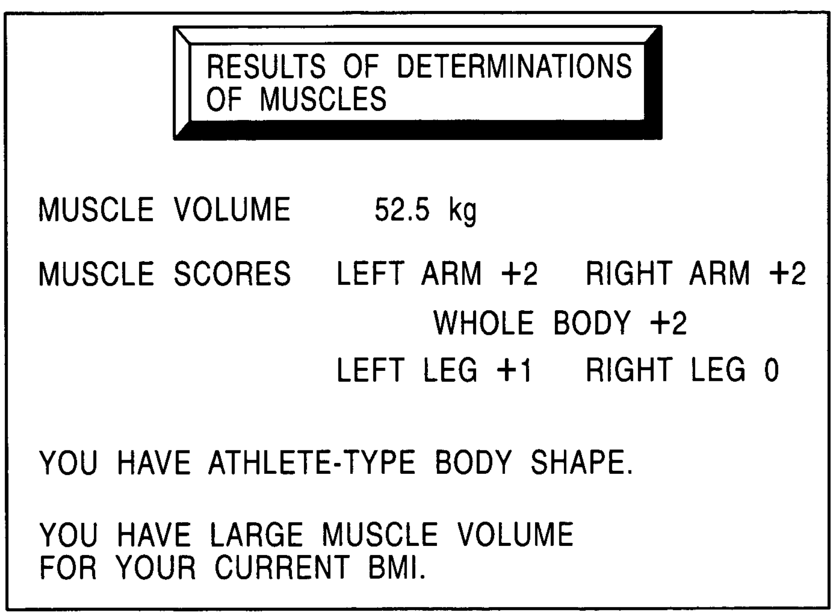
RESULTS OF DETERMINATIONS OF MUSCLES
MUSCLE VOLUME 52.5 kg
MUSCLE SCORES LEFT ARM +2 RIGHT ARM +2
WHOLE BODY +2
LEFT LEG +1 RIGHT LEG 0
YOU HAVE ATHLETE-TYPE BODY SHAPE.
YOU HAVE LARGE MUSCLE VOLUME FOR YOUR CURRENT BMI.

FIG. 8

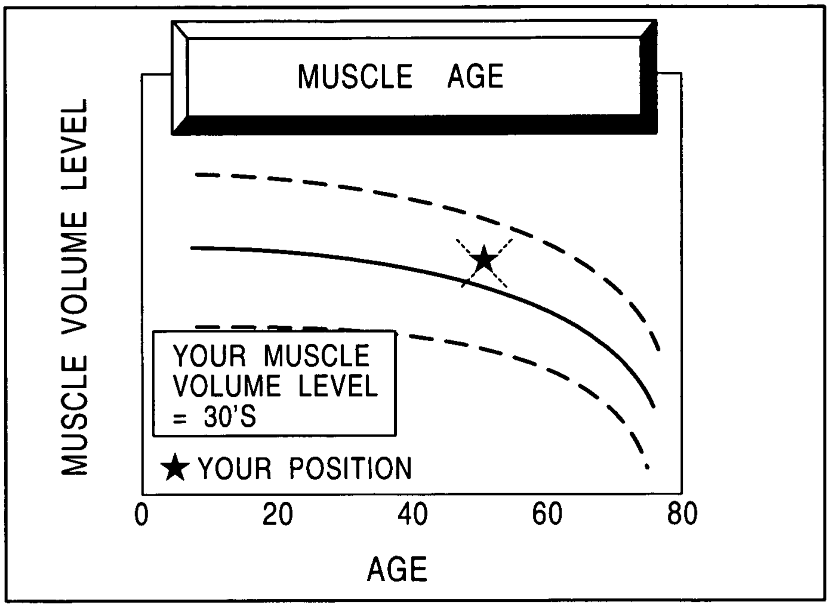
MUSCLE AGE
MUSCLE VOLUME LEVEL
YOUR MUSCLE VOLUME LEVEL = 30'S
★ YOUR POSITION
0
20
40
60
80
AGE

FIG. 9

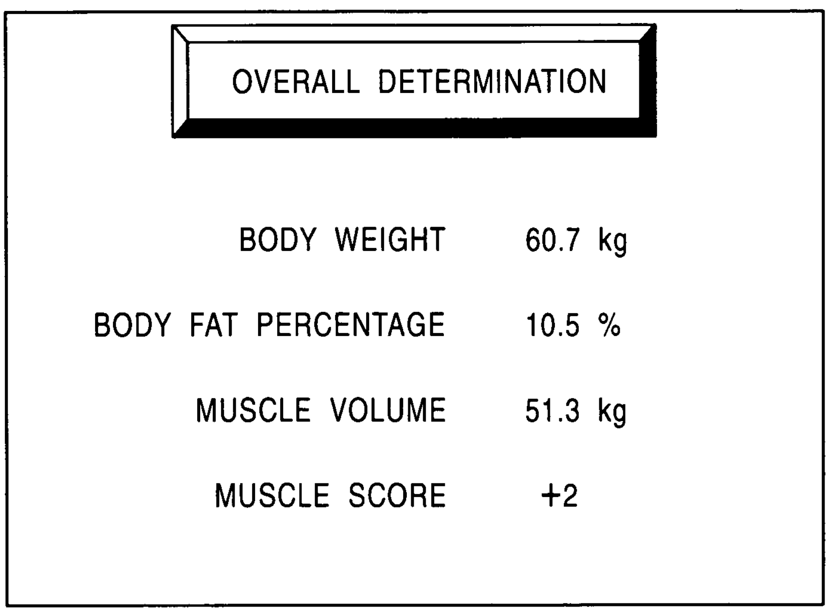
OVERALL DETERMINATION
BODY WEIGHT 60.7 kg
BODY FAT PERCENTAGE 10.5 %
MUSCLE VOLUME 51.3 kg
MUSCLE SCORE +2

European Patent Office

# EUROPEAN SEARCH REPORT

Application Number
EP 04 01 1974

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | EP 1 132 046 A (YAMATO SCALE CO LTD)<br>12 September 2001 (2001-09-12)<br>* column 2, line 5 - line 57 *<br>* column 5, line 22 - column 6, line 3 *<br>* column 6, line 14 - line 22 *<br>* column 8, line 11 - line 22 *<br>* column 9, line 14 - line 26 *<br>* figure 2 *<br>----- | 1-4 | A61B5/053<br>G01G19/414 |
| Y | KYLE U G; GENTON L; HANS D;KARSEGARD V L; MICHEL J P; SLOSMAN D O; PICHARD C: "Total body mass, fat mass, fat-free mass, and skeletal muscle in older people: cross-sectional differences in 60-year-old persons."<br>JOURNAL OF THE AMERICAN GERIATRICS SOCIETY,<br>vol. 49, no. 12, December 2001 (2001-12), pages 1633-1640, XP002294770<br>UNITED STATES<br>* page 1633, right-hand column, line 39 - page 1634, left-hand column, line 4 *<br>* page 1635, left-hand column, line 35 - line 39 *<br>----- | 1-4 | TECHNICAL FIELDS SEARCHED (Int.Cl.7)<br>A61B<br>G01G |
| Y | EP 1 075 858 A (YAMAN LTD)<br>14 February 2001 (2001-02-14)<br>* page 5, line 54 - page 6, line 5 *<br>* figures 5,8,9 *<br>----- | 3 | |
| Y | EP 1 201 187 A (TANITA SEISAKUSHO KK)<br>2 May 2002 (2002-05-02)<br>* column 11, line 24 - line 34 *<br>* column 12, line 7 - line 17 *<br>* figure 10 *<br>----- | 4 | |

The present search report has been drawn up for all claims

| Place of search | Date of completion of the search | Examiner |
|---|---|---|
| The Hague | 2 September 2004 | Quentric, F-X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 04 01 1974

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2004

| Patent document cited in search report | Publication date | Patent family member(s) | Publication date |
|---|---|---|---|
| EP 1132046 A | 12-09-2001 | AU 757258 B2 | 13-02-2003 |
| | | AU 6726900 A | 26-03-2001 |
| | | CA 2348601 A1 | 08-03-2001 |
| | | EP 1132046 A1 | 12-09-2001 |
| | | US 6643542 B1 | 04-11-2003 |
| | | CN 1327376 T | 19-12-2001 |
| | | WO 0115600 A1 | 08-03-2001 |
| | | TW 529930 B | 01-05-2003 |
| EP 1075858 A | 14-02-2001 | JP 2000014828 A | 18-01-2000 |
| | | AU 3537599 A | 16-11-1999 |
| | | EP 1075858 A1 | 14-02-2001 |
| | | WO 9955428 A1 | 04-11-1999 |
| | | JP 2004154589 A | 03-06-2004 |
| | | TW 403669 B | 01-09-2000 |
| EP 1201187 A | 02-05-2002 | JP 2002125947 A | 08-05-2002 |
| | | EP 1201187 A1 | 02-05-2002 |
| | | US 2002049546 A1 | 25-04-2002 |

EPO FORM P0459

For more details about this annex : see Official Journal of the European Patent Office, No. 12/82